(19) **Europäisches Patentamt**
**European Patent Office**
**Office européen des brevets**

(11) **EP 4 076 188 B1**

(12) **EUROPEAN PATENT SPECIFICATION**

(45) Date of publication and mention
of the grant of the patent:
**01.05.2024 Bulletin 2024/18**

(21) Application number: **20829267.2**

(22) Date of filing: **09.12.2020**

(51) International Patent Classification (IPC):
**A61M 25/06** (2006.01)    **A61B 5/15** (2006.01)
**A61B 5/154** (2006.01)

(52) Cooperative Patent Classification (CPC):
**A61B 5/15003; A61B 5/150221; A61B 5/150946;**
**A61B 5/150992; A61B 5/1545;** A61B 5/150732;
A61M 25/0631; A61M 25/0637

(86) International application number:
**PCT/US2020/064125**

(87) International publication number:
**WO 2021/126642 (24.06.2021 Gazette 2021/25)**

(54) **CATHETER EXTENSION SET**

KATHETERVERLÄNGERUNGSSATZ

ENSEMBLE EXTENSION DE CATHÉTER

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **20.12.2019 US 201962951736 P**
**08.12.2020 US 202017114705**

(43) Date of publication of application:
**26.10.2022 Bulletin 2022/43**

(73) Proprietor: **Becton, Dickinson and Company**
**Franklin Lakes, NJ 07417 (US)**

(72) Inventors:
 • **MA, Yiping**
  **Layton, Utah 84040 (US)**
 • **BURKHOLZ, Jonathan Karl**
  **Salt Lake City, Utah 84108 (US)**
 • **BLANCHARD, Curtis H.**
  **Riverton, Utah 84096 (US)**
 • **WANG, Bin**
  **Sandy, Utah 84070 (US)**

(74) Representative: **dompatent von Kreisler Selting**
**Werner -**
**Partnerschaft von Patent- und Rechtsanwälten**
**mbB**
**Deichmannhaus am Dom**
**Bahnhofsvorplatz 1**
**50667 Köln (DE)**

(56) References cited:
**EP-A1- 3 207 970         WO-A1-92/13584**
**WO-A1-92/13584        WO-A1-98/34532**
**WO-A1-2013/028759    WO-A2-2004/032995**
**WO-A2-2019/236956    WO-A2-2019/236956**
**CN-A- 110 141 255      US-A1- 2009 259 201**
**US-A1- 2011 009 717    US-A1- 2014 042 094**
**US-A1- 2019 021 640    US-A1- 2019 321 599**
**US-A1- 2019 321 599**

## Description

<u>BACKGROUND</u>

The present invention relates to a catheter extension set according to the preamble of claim 1 as well as to a catheter extension set according to the preamble of claim 5. Such a catheter extension set is known from WO 2019/236956 A2. A catheter is commonly used to infuse fluids into vasculature of a patient. For example, the catheter may be used for infusing normal saline solution, various medicaments, or total parenteral nutrition. The catheter may also be used for withdrawing blood from the patient.

The catheter may include an over-the-needle peripheral intravenous ("IV") catheter. In this case, the catheter may be mounted over an introducer needle having a sharp distal tip. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe or a test tube with a rubber stopper at one end. In some instances, the blood collection container has had all or a portion of air removed from the test tube so pressure within the blood collection container is lower than ambient pressure. Such a blood collection container is often referred to as an internal vacuum or a vacuum tube. A commonly used blood collection container is a VACUTAINER® blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops.

Unfortunately, as blood is drawn into the blood collection container, red blood cells are in a high shear stress state and susceptible to hemolysis due to a high initial pressure differential between the vein and the blood collection container. Hemolysis may result in rejection and discard of a blood sample. The high initial pressure differential can also result in catheter tip collapse, vein collapse, or other complications that prevent or restrict blood from filling the blood collection container. As the blood collection container fills, a pressure differential between the vein and the blood collection container decreases, and filling of the blood collection tube with blood slows significantly.

The subject matter claimed herein is not limited to embodiments that solve any disadvantages or that operate only in environments such as those described above. Rather, this background is only provided to illustrate one example technology area where some implementations described herein may be practiced.

<u>SUMMARY</u>

The present invention relates to a catheter extension set according to claim 1 as well as to a catheter extension set according to claim 5. In some embodiments, a vascular access system may include the extension set coupled to the vascular access device. In some embodiments, the vascular access device may include a catheter, a needle, or another device suitable for blood collection.

In some embodiments, during blood collection, the extension set may facilitate adjustment of flow resistance within the vascular access system based on a pressure state within the vascular access system. In further detail, in some embodiments, as a pressure differential between a vein of a patient and a blood collection container coupled to the vascular access system changes during blood collection, the extension set may be responsive and may facilitate adjustment of flow resistance within the vascular access system. In some embodiments, the extension set may decrease a risk of hemolysis. In some embodiments, the extension set may simultaneously decrease the risk of hemolysis and decrease blood collection time during a process of blood withdrawal. In some embodiments, the extension set may also reduce a risk of collapse of the vein and/or the vascular access device.

In some embodiments, in response to the pressure differential being high, such as when the blood collection container is first coupled to the vascular access system, the extension set may facilitate increased flow resistance within the vascular access system to distribute the pressure differential and reduce shear stress experienced by red blood cells. In some embodiments, as the blood collection container fills with blood, a vacuum within a fluid pathway of the vascular access system may decrease, and the pressure differential may decrease. Thus, in some embodiments, in order to shorten the blood collection time, the extension set may facilitate decreased flow resistance within the vascular access system while still maintaining the shear stress of the blood cells below a threshold level.

In some embodiments, the extension set may be configured to couple to a catheter assembly and may be referred to as a catheter extension set. According to the invention, the extension set includes a distal end, which includes a luer adapter configured to couple to a catheter adapter or another suitable vascular access device. In some embodiments, the extension set includes a proximal end, which includes a blood collection device. In some embodiments, the blood collection device may include or correspond to a blood collection container. In some embodiments, the blood collection container may include a syringe, an evacuated blood collection tube, a small sample collection device, or any other container configured to collect blood from a patient via a pressure differential. In some embodiments, the blood collection device may include a needle assembly, which may include a needle configured to receive the blood collection container. In these and other embodiments, the blood collection container may include the evacuated blood collection tube.

In some embodiments, the extension set includes an extension tube, which extends between the distal end of the extension set and the proximal end of the extension set. In some embodiments, the extension set may include a clamp, which may be disposed on the extension tube. In some embodiments, the clamp may be configured to move between a clamped position and an unclamped position. In some embodiments, the clamp may prevent or reduce fluid flow through the extension tube in response to the clamp being in the clamped position.

In some embodiments, the luer adapter may be a first luer adapter. In some embodiments, the blood collection device may include a second luer adapter. In some embodiments, the extension set may include a third luer adapter, which may be coupled to the second luer adapter. In some embodiments, the extension tube may include a distal end and a proximal end. In some embodiments, the distal end of the extension tube may be integrated with the first luer adapter. In some embodiments, the proximal end of the extension tube may be integrated with the third luer adapter. In other embodiments, the proximal end of the extension tube may be integrated with the blood collection device.

According to the present invention, an inner surface of the extension tube includes an absorbent material or an indicator tube comprising a plurality of markers, wherein blood within the extension tube is configured to flow proximate the absorbent material or through the indicator tube. In some embodiments, in response to blood reaching or saturating a particular marker, a clinician may change the flow resistance within the vascular access system via the extension set. In some embodiments, to decrease the flow resistance within the vascular access system, the clinician may remove the extension set from the vascular access system, move the clamp to the unclamped position, or perform another action to decrease the flow resistance as may be further described in the present disclosure.

According to the present invention, the extension set includes a first extension tube and a second extension tube. In some embodiments, a flow resistance of the first extension tube may be greater than a flow resistance of the second extension tube. For example, the first extension tube may be longer than the second extension tube. Additionally or alternatively, in some embodiments, an inner diameter of the first extension tube may be less than an inner diameter of the second extension tube. In some embodiments, the flow resistance of the first extension tube may be equal to the flow resistance of the second extension tube. In some embodiments, the second extension tube may include any combination of length and inner diameter such that the flow resistance of the second extension tube is the same or less than the flow resistance of the first extension tube.

According to the present invention, the first extension tube and the second extension tube extend between the distal end of the extension set and the proximal end of the extension set. In some embodiments, the clamp may be disposed on the second extension tube. In some embodiments, the second extension tube may extend through the clamp. According to the present invention, an inner surface of the first extension tube and/or the second extension tube include an absorbent material or an indicator tube comprising a plurality of markers, wherein blood within the first extension tube and/or the second extension tube is configured to flow proximate the absorbent material or through the indicator tube.

In some embodiments, the first extension tube may be disposed within the second extension tube. In some embodiments, in response to the clamp being in the clamped position, blood may flow through the first extension tube, and blood flow between an outer surface of the first extension tube and an inner surface of the second extension tube may decrease or stop. In some embodiments, to decrease or stop the flow resistance within the vascular access system after the blood collection container is partially filled and the pressure differential has decreased, the clinician may move the clamp to the unclamped position to increase a flow rate given the pressure differential has decreased.

In some embodiments, the second extension tube may include a distal end and a proximal end. In some embodiments, the distal end of the first extension tube and the distal end of the second extension tube may be integrated with the first luer adapter. In some embodiments, the proximal end of the first extension tube and the proximal end of the second extension tube may be integrated with the third luer adapter.

In some embodiments, in response to the second luer adapter being tightened with respect to the third luer adapter, a first fluid pathway through the first extension tube may be open, and a second fluid pathway through the second extension tube may be closed. In some embodiments, in response to the second luer adapter being loosened with respect to the third luer adapter, the first fluid pathway through the first extension tube may be open, and the second fluid pathway through the second extension tube may be open. In some embodiments, to decrease the flow resistance within the vascular access system after the blood collection container is partially filled and the pressure differential has decreased, the clinician may loosen the second luer adapter with respect to the third luer adapter or move the second luer adapter from a tightened position to a loosened position. As a result, in these and other embodiments, a flow rate may increase.

In some embodiments, the extension set may include a pressure-sensitive valve within the extension set. In some embodiments, the pressure-sensitive valve may be disposed in the second extension tube. In some embodiments, the pressure-sensitive valve may be disposed in a portion of the fluid pathway of the extension set distal to the first extension tube and the second extension tube. In some embodiments, the fluid pathway of the extension set may extend through the proximal end of the extension set and the distal end of the extension set. In some embodiments, the fluid pathway of the extension set may include the first fluid pathway of the first extension tube and/or the second fluid pathway of the second extension tube. In some embodiments, the pressure-sensitive valve may be disposed at a junction of the first fluid pathway and the second fluid pathway.

In some embodiments, in response to a first predetermined pressure differential within the extension set, the pressure-sensitive valve may be open with respect to the first extension tube and closed with respect to the second extension tube. In some embodiments, in response to a second predetermined pressure differential within the extension set, the pressure-sensitive valve may be closed with respect to the first extension tube and open with respect to the second extension tube. In some embodiments, the first predetermined pressure differential may be greater than the second predetermined pressure differential. In some embodiments, the first predetermined pressure differential and the second predetermined pressure differential may correspond to the pressure differential between the vein of the patient and the blood collection container coupled to the vascular access system.

In some embodiments, a not claimed method of blood collection or collecting a blood sample may include inserting a catheter of a catheter system into vasculature of the patient. In some embodiments, the catheter system may include a catheter assembly, which may include a catheter adapter and the catheter. In some embodiments, the catheter may be secured within the catheter adapter and may extend distally from the catheter adapter.

In some embodiments, the method of blood collection may include coupling the extension set to the catheter adapter. In some embodiments, the method of blood collection may include blocking blood flow through the second extension tube. In some embodiments, after inserting the catheter of the catheter system into the vasculature of the patient, coupling the catheter extension set to the catheter adapter, and blocking blood flow through the second extension tube, the method of blood collection may include coupling a blood collection container to the blood collection device or the third luer adapter. In some embodiments, in response to coupling the blood collection container to the blood collection device or the third luer adapter, the blood collection container may begin to fill with blood. In some embodiments, in response to the blood collection container filling partially with blood, the method of blood collection may include opening the second extension tube such that increased blood flows through the second extension tube.

In some embodiments, the second extension tube may extend through the clamp. In these embodiments, blocking blood flow through the second extension tube may include moving the clamp to the clamped position, and opening the second extension tube may include moving the clamp to the unclamped position. In some embodiments, blocking blood flow through the second extension tube may include tightening the second luer with respect to the third luer adapter. In some embodiments, opening the second extension tube may include loosening the second luer with respect to the third luer adapter.

In some embodiments, the extension set may include a roller clamp. In some embodiments, the roller clamp may include a roller moveable within a track. In some embodiments, the first extension tube and the second extension tube may extend through the roller clamp generally perpendicular to the track. In some embodiments, when blood flow through the second extension tube is blocked, the roller may be disposed in a first position proximate the second extension tube. In some embodiments, opening the second extension tube may include moving the roller along the track away from the second extension tube and toward the first extension tube.

In some embodiments, the distal end of the extension set may include a three-way stopcock, which may include a first port, a second port, and a third port. In some embodiments, the first extension tube may be coupled with the first port, the second extension tube may be coupled to the second port, and the third port may include the first luer adapter configured to couple to the catheter adapter. In some embodiments, blocking blood flow through the second extension tube may include rotating the three-way stopcock to a first position. In some embodiments, opening the second extension tube may include rotating the three-way stopcock to a second position.

It is to be understood that both the foregoing general description and the following detailed description are examples and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

BRIEF DESCRIPTION OF THE SEVERAL VIEWS OF THE DRAWINGS

Example embodiments will be described and explained with additional specificity and detail through the use of the accompanying drawings in which:

Figure 1A is an upper perspective view of an example extension set, according to some embodiments;

Figure 1B is a cross-sectional view of the extension set of Figure 1A, according to some embodiments;

Figure 1C is an upper perspective view of the extension set of Figure 1A coupled to an example catheter assembly, according to some embodiments;

Figure 2A is an upper perspective view of another example extension set, according to some embodiments;

Figure 2B is a cross-sectional view of the extension set of Figure 2B, according to some embodiments;

Figure 2C is an upper perspective view of the extension set of Figure 2A coupled to the catheter assembly of Figure 1C, according to some embodiments;

Figure 3A is an upper perspective view of another example extension set, according to some embodiments;

Figure 3B is a cross-sectional view of the extension set of Figure 3A coupled to another example catheter assembly, according to some embodiments;

Figure 3C is an upper perspective view of the extension set of Figure 3A coupled to the catheter assembly of Figure 3B, according to some embodiments;

Figure 4A is an upper perspective view of another example extension set, according to some embodiments;

Figure 4B is an upper perspective view of the extension set of Figure 4A coupled to the catheter assembly of Figure 3B, according to some embodiments;

Figure 4C is a cross-sectional view of the extension set of Figure 4A coupled to the catheter assembly of Figure 3B, according to some embodiments;

Figure 5A is a schematic diagram of the extension set of Figure 3A, illustrating an example clamp in a clamped position prior to entry of blood into the extension set, according to some embodiments;

Figure 5B is a schematic diagram of the extension set of Figure 3A, illustrating blood flowing through an example first extension tube under a high initial pressure differential, according to some embodiments;

Figure 5C is a schematic diagram of the extension set of Figure 3A, illustrating the clamp removed or unclamped in response to a lower pressure differential, according to some embodiments;

Figure 6A is a cross-sectional view of an example second luer adapter and an example third luer adapter prior to being coupled together, according to some embodiments;

Figure 6B is a cross-sectional view of the second luer adapter and the third luer adapter of Figure 6A, illustrating the second luer adapter tightened with respect to the third luer adapter, according to some embodiments;

Figure 6C is a cross-sectional view of the second luer adapter and the third luer adapter of Figure 6A, illustrating the second luer adapter loosened with respect to the third luer adapter, according to some embodiments;

Figure 7A is an upper perspective view of an example roller clamp that may be used with the extension set of Figure 3A or the extension set of Figure 4A, according to some embodiments;

Figure 7B is a cross-sectional view of the roller clamp of Figure 7A, illustrating an example roller in an example first position, according to some embodiments;

Figure 7C is a cross-sectional view of the roller clamp of Figure 7B, illustrating the roller in an example second position, according to some embodiments;

Figure 8 is a cross-sectional view of another example extension set coupled to the catheter assembly of Figure 3B, according to some embodiments;

Figure 9A is an upper perspective view of another example extension set coupled to the catheter assembly of Figure 3B, according to some embodiments;

Figure 9B is an enlarged upper perspective view of a portion of the extension set of Figure 9A, according to some embodiments;

Figure 9C is a cross-sectional view of the extension set of Figure 9A, according to some embodiments;

Figure 9D is a transverse cross-sectional view of the extension set of Figure 9A, illustrating another example first extension tube disposed within an example second extension tube and the second extension tube clamped, according to some embodiments;

Figure 9E is a transverse cross-sectional view of the extension set of Figure 9A, illustrating the first extension tube disposed within the second extension tube, according to some embodiments;

Figure 10A is a cross-sectional view of a portion of another example first extension tube, illustrating an example absorbent material prior to entry of blood into the first extension tube, according to some embodiments;

Figure 10B is a cross-sectional view of the portion of the first extension tube of Figure 10A, illustrating the absorbent material in response to blood flowing through the first extension tube under a high initial pressure differential, according to some embodiments;

Figure 11A is a cross-sectional view of a portion of another example first extension tube, illustrating an example indicator tube prior to entry of blood into the first extension tube, according to some embodiments;

Figure 11B is a cross-sectional view of the portion of the first extension tube of Figure 11A, illustrating the indicator tube in response to blood flowing through the first extension tube under the high initial pressure differential, according to some embodiments;

Figure 12A is a cross-sectional view of an example junction between another example first extension tube and an example second extension tube, illustrating the junction prior to entry of blood into the junction, according to some embodiments;

Figure 12B is a cross-sectional view of the junction of Figure 12A, illustrating the junction in response to blood flowing through the junction under the high initial pressure differential, according to some embodiments;

Figure 12C is a cross-sectional view of the junction of Figure 12A, illustrating the junction in response to blood flowing through the junction under a lower pressure differential compared to the high initial pressure differential, according to some embodiments;

Figure 13 is a partial cutaway view of another example extension set coupled to the catheter assembly of Figure 3B, according to some embodiments;

Figure 14A is an upper perspective view of an example catheter system, according to some embodiments; and

Figure 14B is an upper perspective view of another example catheter system, according to some embodiments.

## DESCRIPTION OF EMBODIMENTS

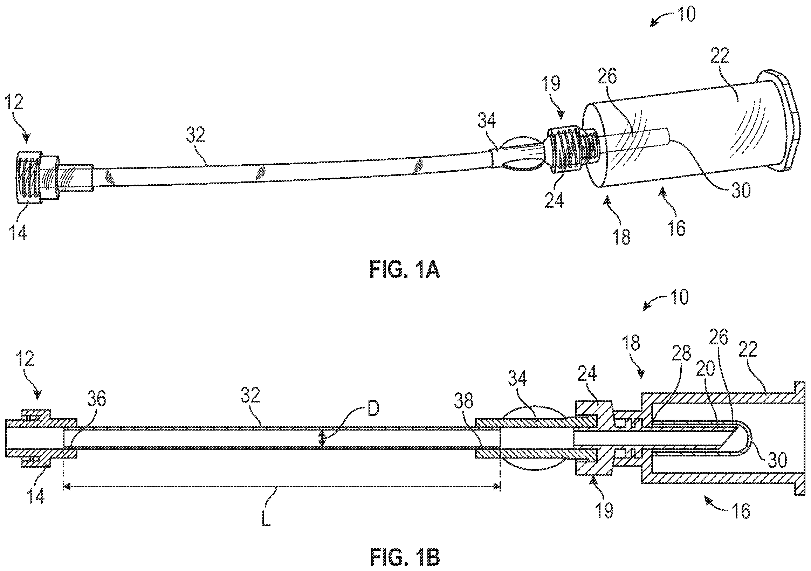

Referring now to Figures 1A-1C, in some embodiments, an extension set 10 may include a distal end 12, which may include a luer adapter 14 configured to couple to a catheter adapter or another suitable vascular access device. In some embodiments, the extension set 10 may include a proximal end 16, which may include a blood collection device 18. In some embodiments, the blood collection device 18 may include or correspond to a blood collection container. In some embodiments, the blood collection container may include a syringe, an evacuated blood collection tube, a small sample collection device, or any other container configured to collect blood from a patient via a pressure differential.

In some embodiments, the blood collection device 18 may include a needle assembly 19, which may include a needle 20 configured to receive a blood collection container. In these and other embodiments, the blood collection container may include the evacuated blood collection tube. In some embodiments, the blood collection container has all or a portion of air removed so pressure within the blood collection container is lower than ambient pressure.

In some embodiments, the needle assembly 19 may include one or more threads, which may be configured to couple to a holder 22, which may be generally cylindrical and may be configured to hold the blood collection container. In some embodiments, the holder 22 may be integrally formed with the needle assembly 19 or coupled to the needle assembly 19 via bonding or another suitable method. In some embodiments, the holder 22 may surround the needle 20. In some embodiments, the needle assembly 19 and the holder 22 may include or correspond to a luer lock access device, such as, for example, the VACUTAINER® LUER-LOK™ ACCESS DEVICE available from Becton Dickinson & Company. In some embodiments, the holder 22 may include or correspond to the blood collection tube holder 127 described in U.S. Patent Application No. 62/928,69, filed October 30, 2019, entitled "BLOOD COLLECTION SYSTEM WITH USER-ADJUSTED PRESSURE MANAGEMENT AND RELATED METHODS,"

In some embodiments, the luer adapter 14 may be a first luer adapter 14. In some embodiments, the extension set 10 may include a second luer adapter 24. In some embodiments, the blood collection device 18 may include the second luer adapter 24. In some embodiments, the needle 20 may be integrated with the second luer adapter 24. In some embodiments, a proximal end of the needle 20 may be enveloped within an elastomeric sheath 26. In some embodiments, the elastomeric sheath 26 may include an open distal end 28 and a closed proximal end 30. In some embodiments, in response to the blood collection container pushing the elastomeric sheath 26 distally, the needle 20 may pierce the elastomeric sheath 26, and the needle 20 may insert into a cavity of the blood collection container.

In some embodiments, the extension set 10 may include an extension tube 32, which may extend between the distal end 12 of the extension set 10 and the proximal end 16 of the extension set 10. In some embodiments, the extension tube 32 may be rigid or semi-rigid, which may reduce a likelihood of kinking.

In some embodiments, the extension set 10 may include a third luer adapter 34, which may be coupled to the second luer adapter 24. In some embodiments, the extension tube 32 may include a distal end 36 and a proximal end 38. In some embodiments, the distal end 36 may be coupled to or integrated with the first luer adapter 14. In some embodiments, the proximal end of the extension tube 32 may be coupled to or integrated with the third luer adapter 34. In other embodiments, the proximal end of the extension tube 32 may be coupled to or integrated with the blood collection device 18.

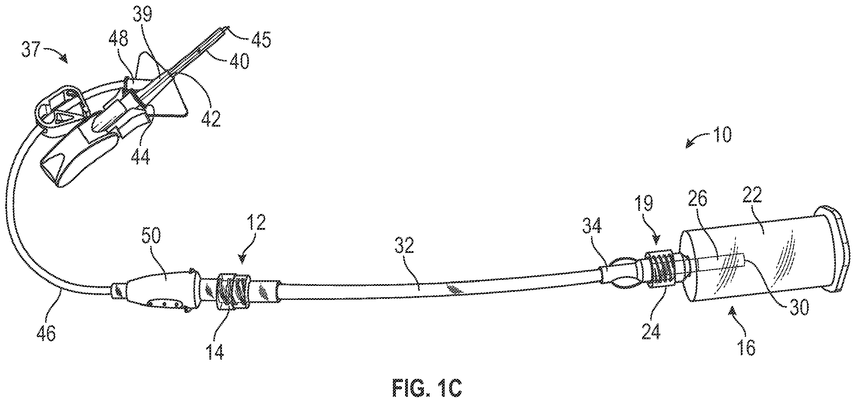

In some embodiments, the extension set 10 may act as a flow resistor in a fluid pathway of a catheter system, illustrated, for example, in Figure 1C, or another vascular access system. In some embodiments, the catheter system may include a catheter assembly 37, which may include a catheter adapter 39 and a catheter 40. In some embodiments, the catheter 40 may be secured within the catheter adapter 39 and may extend distally from the catheter adapter 39. In some embodiments, the catheter adapter 39 may include a distal end 42, a proximal end 44, and a lumen extending through the distal end 42 and the proximal end 44. In some embodiments, an introducer needle 45 may extend from a needle shield through the catheter 40.

In some embodiments, the extension set 10 may be coupled to the catheter assembly 37 in any number of suitable ways. In some embodiments, the catheter assembly 37 may be integrated. In further detail, in some embodiments, another extension tube 46 may extend from a side port 48 the catheter adapter 39. In some embodiments, a proximal end of the other extension tube 46 may include a fourth luer adapter 50, which may be coupled to the first luer adapter 14. In some embodiments, the catheter assembly 37 may be straight and/or the first luer adapter 14 may be coupled to the proximal end 44 of the catheter adapter 39. In some embodiments, one or more of the first luer adapter 14, the second luer adapter 24, the third luer adapter 34, and the fourth luer adapter 50 may include a slip or thread or clip male luer adapter, a slip or thread female luer adapter, a needleless connector, a blunt cannula, or another suitable access device.

In some embodiments, the catheter assembly 37 may include or correspond to any suitable catheter assembly, such as, for example, the BD NEXIVA™ Closed IV Catheter system, the BD CATHENA™ Catheter system, the BD VENFLON™ Pro Safely Shielded IV Catheter system, the BD NEOFLON™ IV Cannula system, the BD INSYTE™ AUTOGUARD™ BC Shielded IV Catheter system, or another suitable catheter assembly. In some embodiments, the catheter 40 may include a peripheral intravenous catheter (PIVC), a peripherally inserted central catheter (PICC), or a midline catheter.

In some embodiments, the fluid pathway of the catheter system may include one or more of the following: the catheter 40, the catheter adapter 39, the other extension tube 46, the fourth luer adapter 50, the first luer adapter 14, the extension tube 32, the third luer adapter 34, the second luer adapter 24, and the blood collection device 18. In some embodiments, the extension set 10 may lower a flow rate of blood within the fluid pathway of the catheter system, which may in turn lower a shear rate for hemolysis management. In some embodiments, the catheter assembly 37 may be substituted with another type of vascular access device, such as, for example, a venipuncture device, an infusion disposable, a blood collection access device, or a blood collection container.

Blood cell experiences shear stress as it flows in a fluid pathway. The maximum shear stress is along the wall of the fluid pathway, or wall shear stress. Wall shear stress on blood cells is considered a major source of mechanical damage to blood cells. For a cylindrical fluid path, the wall shear stress is typically expressed as:

$$\tau = \frac{1}{2} \cdot \frac{\Delta p}{L} \cdot (kr)$$

in which $\Delta P$ is the pressure drop along a path with a length of $L$ and an interior radius of $r$. $k$ is shrinkage index.

To fill a certain volume of collection tube, $V$, with a flow rate of $Q$, the time needed can be simply assessed by:

$$t = \frac{v}{Q} = 8\mu v \cdot \frac{1}{\pi r^4} / \left(\frac{\Delta p}{L}\right)$$

in which $\mu$ is the dynamic viscosity of the fluid. Hemolysis is typically associated with both the wall shear stress and the time a blood cell is exposed to wall shear stress. From literature, it has been widely considered that hemolysis index can be approached as a function of:

$$HI(\%) = A * t^{\alpha} * \tau^{\beta}$$

in which $A$, $\alpha$, and $\beta$ are coefficients.

In principle, the hemolysis index is related to pressure gradient and cross-sectional characteristic dimension:

$$HI(\%) \propto \left(\frac{\Delta P}{l}\right)^{\beta - \alpha} \cdot \left(\frac{1}{r}\right)^{4\alpha - \beta}$$

In some embodiments, a length of the extension tube 32 may be selected based on one or more of the following: a gauge and/or length of a particular catheter, a particular catheter assembly configuration, or a clinical setup. In some embodiments, the extension tube 32 may include a length L from the first luer adapter 14 to the second luer adapter 24. In some embodiments, the extension tube 32 may include an inner diameter D.

Fluid flow in an extension tube with a tubular fluid pathway therethrough can be analyzed using Poiseuille's equation:

$$Q = \frac{\pi D^4 \Delta P}{128 \mu L} = \frac{\Delta P}{R_f}$$

where $\Delta P$ is a change in pressure gradient across the length of the extension tube, D and L are the inner diameter and length, respectively, of the tubular fluid pathway through the extension tube, $\mu$ is the viscosity of a fluid,

and $R_f = \dfrac{128\mu L}{\pi D^4}$ is the fluid resistance. The extension tube may include or correspond to the extension tube 32. Since $\mu$ is the viscosity of the fluid and not part of the extension tube geometry, a geometric factor $G_f$ is defined such that $R_f$ (the fluid resistance) is $R_f = \dfrac{128\mu}{\pi} G_f$ ,

where $G_f = \dfrac{L}{D^4}$ .

In some embodiments, the extension tube 32 may have multiple sections with lengths (L1, L2, L3) and inner diameters of (D1, D2, D3), the geometric factor is then:

$$G_f = \frac{L1}{D1^4} + \frac{L2}{D2^4} + \frac{L3}{D3^4}$$

In some embodiments, the extension tube 32 may have an inner diameter that changes over the length of the extension tube, the geometric factor is then:

$$G_f = \int_0^L \frac{dl}{D(l)^4}$$

In some embodiments, the extension tube 32 may have a cross section that is not circular. In this case, the geometric factor can be determined by measuring the flow rate (Q) at given pressure ($\Delta P$) with known viscosity ($\mu$) fluid:

$$G_f = \frac{\pi \Delta P}{128\mu Q}$$

The $G_f$ value may be selected to reduce the max shear stress for each catheter gauge to be the same or less than the max shear stress of a 21G VACUTAINER® UTLRATOUCH™ push button blood collection set (available from Becton Dickinson & Company), which was previously considered the gold standard for blood draws. In some embodiments, $G_f$ may be equal to or more than 3.83E+06 (1/in$^3$) when a 18G catheter is used, which may reduce the wall sheer stress to reduce hemolysis. In some embodiments, $G_f$ may be equal to or more than 3.27E+06 (1/in$^3$) when a 20G catheter is used, which may reduce the wall sheer stress to reduce hemolysis. In some embodiments, $G_f$ may be equal to or more than 3.33E+06 (1/in$^3$) when a 22G catheter is used, which may reduce the wall sheer stress to reduce hemolysis. In some embodiments, $G_f$ may be equal to or more than 1.50E+07 (1/in$^3$) when a 24G catheter is used, which may reduce the wall sheer stress to reduce hemolysis. In some embodiments, $G_f$ may include another value. In some embodiments, $G_f$ value may be selected to reduce the max shear stress for each catheter gauge to be the same or less than the max shear stress of a 25G VACUTAINER® ULTRATOUCH™ push button blood collection set (available from Becton Dickinson & Company).

In some embodiments, when a 18G catheter is used, $G_f$ may be equal to 3.83E+06 (1/in$^3$) plus or minus 10 percent, plus or minus 25 percent, plus or minus 50 percent, or plus or minus 75 percent, which may reduce the wall sheer stress to reduce hemolysis. In some embodiments, when a 20G catheter is used, $G_f$ may be equal to 3.27E+06 (1/in$^3$) plus or minus 10 percent, plus or minus 25 percent, plus or minus 50 percent, or plus or minus 75 percent, which may reduce the wall sheer stress to reduce hemolysis. In some embodiments, when a 22G catheter is used, $G_f$ may be equal to 3.33E+06 (1/in$^3$) plus or minus 10 percent, plus or minus 25 percent, plus or minus 50 percent, or plus or minus 75 percent, which may reduce the wall sheer stress to reduce hemolysis. In some embodiments, when a 24G catheter is used, $G_f$ may be equal to 1.50E+07 (1/in$^3$) plus or minus 10 percent, plus or minus 25 percent, plus or minus 50 percent, or plus or minus 75 percent, which may reduce the wall sheer stress to reduce hemolysis. In some embodiments, $G_f$ may include another value, which may be selected based on a gauge of the catheter. In some embodiments, $G_f$ values may be selected to be the same for 22G through 18G catheters.

In some embodiments, the fluid pathway of the catheter system, which may include one or more of the needle assembly 19, the extension tube 32, and the catheter assembly 37 (which may include the other extension tube 46), may include an entirety of a blood collection pathway through which blood flows during blood collection. The system geometric factor $G_{fs}$ for the fluid pathway of the catheter system can be determined in similar fashion as described earlier. In some embodiments, the system geometric factor $G_{fs}$ may be equal to or more than 7.34E+06 (1/in$^3$). In some embodiments, $G_{fs}$ may include another value. In some embodiments, the system geometric factor $G_{fs}$ may be 7.34E+06 (1/in$^3$) plus or minus 10 percent, plus or minus 25 percent, plus or minus 50 percent, or plus or minus 75 percent. In some embodiments, $G_{fs}$ may include another value, which may be selected based on a gauge of the catheter.

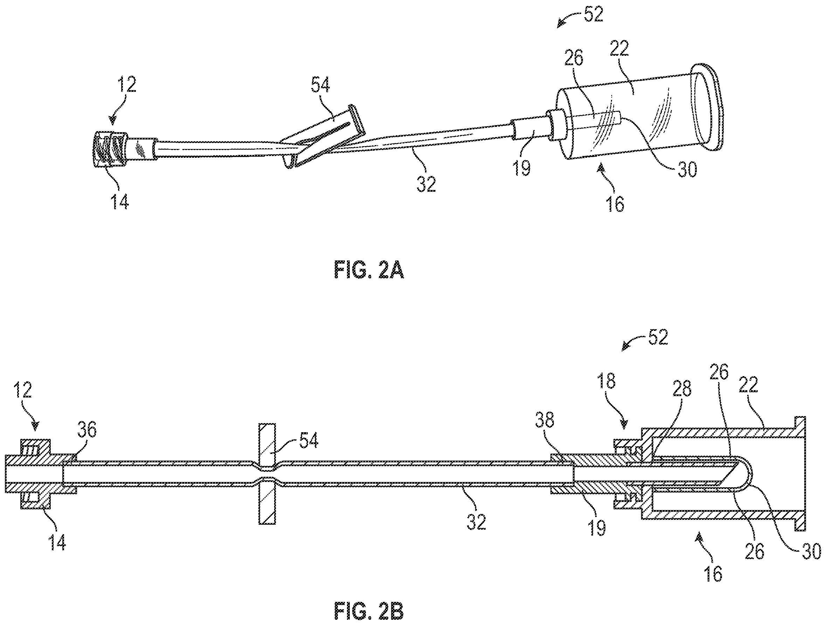

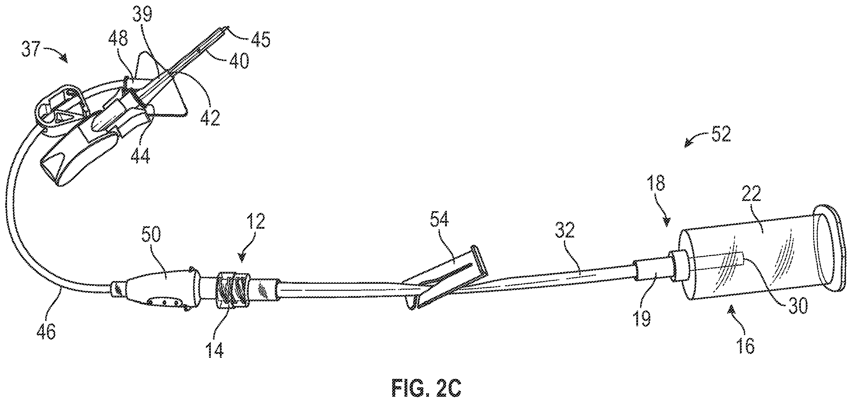

Referring now to Figures 2A-2C, an extension set 52 is illustrated, according to some embodiments. In some embodiments, the extension set 52 may be similar or identical to the extension set 10 of Figures 1A-1C in terms of one or more included features and/or operation. In some embodiments, the proximal end of the extension tube 32 may be integrated with the blood collection device 18. In some embodiments, the extension set 52 may include a clamp 54, which may be disposed on the extension tube 32. In some embodiments, the clamp 54 may be configured to move between a clamped position and an unclamped position or between a more clamped position and a less clamped position. In some embodiments, the clamp 54 may prevent or reduce fluid flow through the extension tube 32 in response to the clamp 54 being in the clamped position.

In some embodiments, the clinician may adjust flow resistance within the catheter system by manually changing fluid characteristics of the catheter system via the clamp 54. In some embodiments, in response to the clamp 54 being in the clamped position, flow resistance within the catheter system may be increased and blood flow through the extension tube 32 may be reduced. In these embodiments, a risk of hemolysis may be reduced. In some embodiments, to decrease the flow resistance within the catheter system after the blood collection container nears filling, the clinician may move the clamp to the unclamped position, which may allow faster blood collection when a risk of hemolysis is reduced.

In some embodiments, the clamp 54 may include a slide clamp, which may include a slot that becomes progressively narrower. In these and other embodiments, the extension tube 32 may be flexible and compliant. In some embodiments, the clinician may adjust an inner diameter of the extension tube 32 by adjusting a depth of the extension tube 32 within the slot of the slide clamp. The clinician may in turn adjust a flow resistance within the extension set 52. In some embodiments, the clamp 54 may include a roller clamp, a slide clamp, a pinch clamp, or another suitable type of clamp.

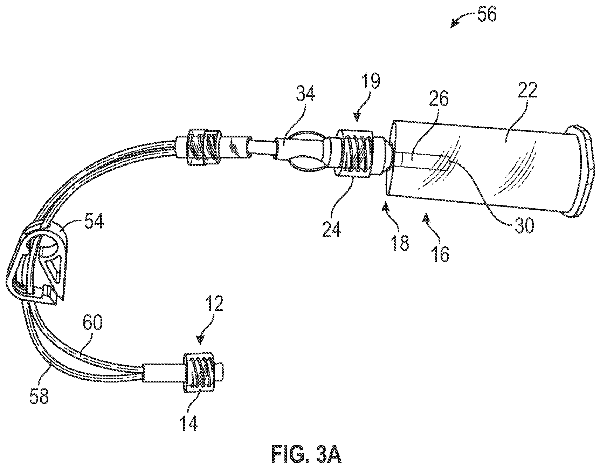

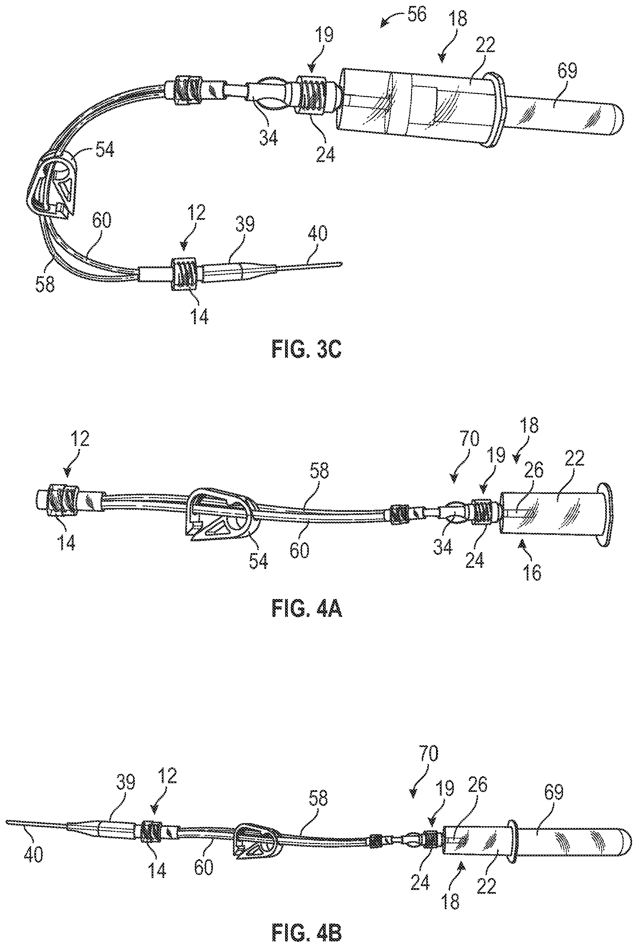

Referring now to Figures 3A-3C, an extension set 56 is illustrated, according to some embodiments. In some embodiments, the extension set 56 may be similar or identical to the extension set 10 of Figures 1A-1C and/or the extension set 52 in terms of one or more included features and/or operation. In some embodiments, the extension set 56 may include one or more extension tubes. In some embodiments, the extension tube 32 may include or correspond to a first extension tube 58 of the extension set 56. In some embodiments, the extension set 56 may include a second extension tube 60.

It is understood that in some embodiments, any suitable first lumen may be substituted for the first extension tube 58 and/or any suitable second lumen may be substituted for the second extension tube 60. Thus, in some embodiments, a particular extension set may not include the first extension tube 58 and/or the second extension tube 60 and may instead include the first lumen and/or the second lumen. In some embodiments, the first lumen and the second lumen may be disposed in a single multi-lumen extension tube or any other suitable structure. In some embodiments, a distal end of the single multi-lumen extension tube may be coupled to or integrated with the first luer adapter 14. In some embodiments, a proximal end of the single multi-lumen extension tube may be coupled to or integrated with the third luer adapter 34. In some embodiments, a proximal end of the single multi-lumen extension tube may be coupled to or integrated with the blood collection device 18.

In some embodiments, a flow resistance of the first extension tube 58 may be greater than a flow resistance of the second extension tube 60. For example, the first extension tube 58 may be longer than the second extension tube 60, as illustrated, for example, in Figures 3A-3C. Additionally or alternatively, in some embodiments, an inner diameter of the first extension tube 58 may be less than an inner diameter of the second extension tube 60, as illustrated, for example, in Figures 4A-4C. In some embodiments, the flow resistance of the first extension tube 58 may be equal to the flow resistance of the second extension tube 60. In some embodiments, the second extension tube 60 may include any combination of length and inner diameter such that the flow resistance of the second extension tube 60 is the same or less than the flow resistance of the first extension tube 58.

In some embodiments, the first extension tube 58 and the second extension tube 60 may extend between a distal end 12 of the extension set 56 and a proximal end 16 of the extension set 56. In some embodiments, the proximal end 16 of the extension set may include the blood collection device 18 or the third luer connector 34. In some embodiments, a clamp 54 may be disposed on the second extension tube 60. In some embodiments, the second extension tube 60 may extend through the clamp 54.

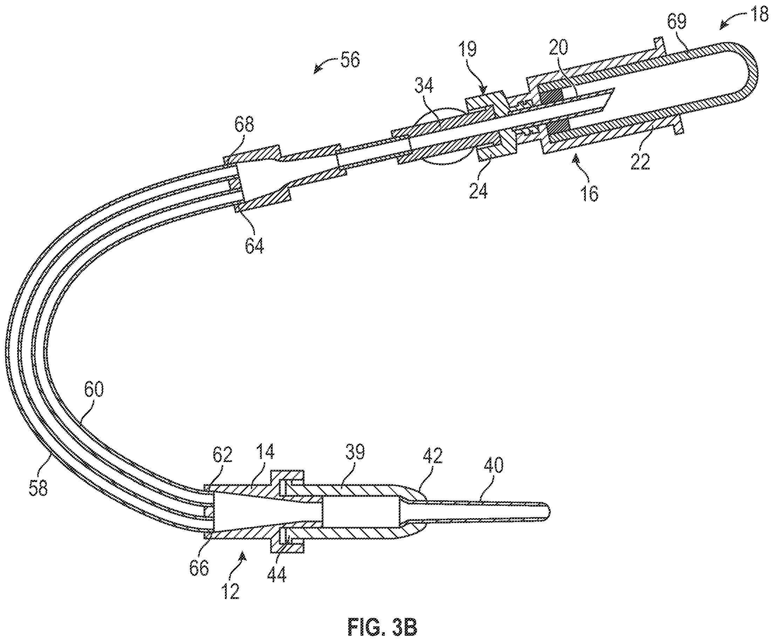

In some embodiments, the second extension tube 60 may include a distal end 62 and a proximal end 64. In some embodiments, a distal end 66 of the first extension tube 58 and the distal end 62 of the second extension tube 60 may be integrated with the first luer adapter 14, as illustrated, for example, in Figure 3B. In some embodiments, a proximal end 68 of the first extension tube 58 and the proximal end 64 of the second extension tube 60 may be integrated with the third luer adapter 34 or there may be one or more components between the proximal ends 64, 68 and the third luer adapter 34, such as, for example, a short tubing. In some embodiments, the proximal end 68 of the first extension tube 58 and the proximal end 64 of the second extension tube 60 may be integrated with the blood collection device 18.

In some embodiments, in response to the clamp 54 being in the clamped position, flow resistance may be increased as blood may flow through the first extension tube 58. In some embodiments, in response to the clamp 54 being in the clamped position, blood flow through the second extension tube 60 may be reduced or nothing. In some embodiments, to decrease the flow resistance after the blood collection container is partially filled and the pressure differential has decreased, the clinician may move the clamp 54 to the unclamped position, which may allow faster blood collection. An example blood collection container 69 is illustrated in Figures 3B-3C.

Referring now to Figures 4A-4C, an extension set 70 is illustrated, according to some embodiments. In some embodiments, the extension set 70 may be similar or identical to one or more of the following in terms of one or more included features and/or operation: the extension set 10 of Figures 1A-1C, the extension set 52 of Figures 2A-2C, and the extension set 56 of Figures 3A-3C. In some embodiments, an inner diameter of the first extension tube 58 may be less than an inner diameter of the second extension tube 60. In some embodiments, the first extension tube 58 and the second extension tube 60 may be a same length.

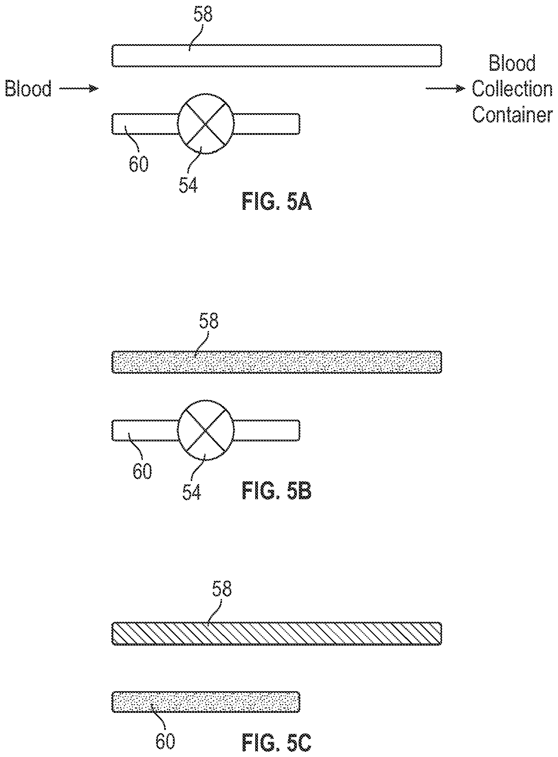

Referring now to Figures 5A-5C, a schematic diagram of an extension set having multiple extension tubes is illustrated, according to some embodiments. The extension set may correspond to the extension set 56 or the extension set 70, for example. In some embodiments, as illustrated in Figure 5A, the clamp 54 may be in the clamped position prior to entry of blood into the extension set. In some embodiments, as illustrated in Figure 5B, blood may flow through the first extension tube 58 in response to the second extension tube 60 being clamped. In some embodiments, as illustrated in Figure 5C, in response to the clamp being removed, unclamped, or less clamped, increased blood may flow through the second extension tube 60, which may increase a total amount of blood flowing through the extension set.

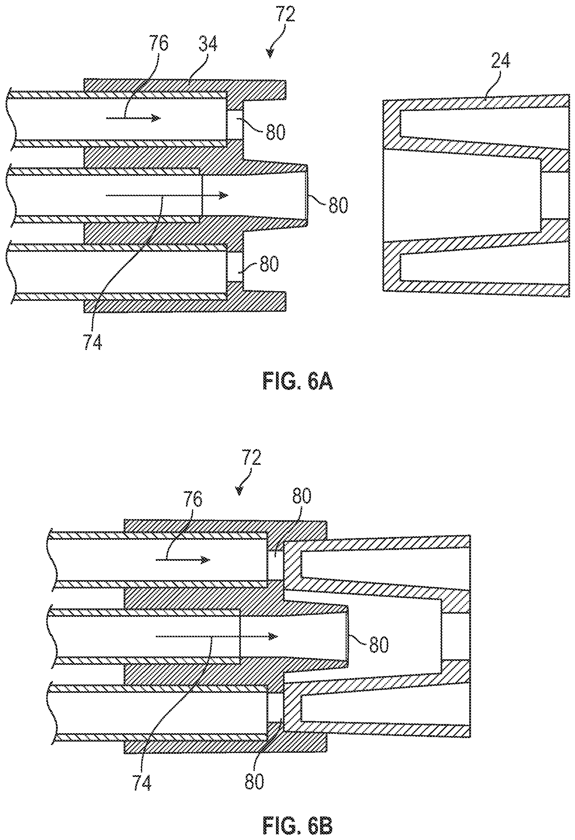

Referring now to Figures 6A-6C, a portion of an extension set 72 is illustrated, according to some embodiments. In some embodiments, the extension set 72 may be similar or identical to one or more of the following in terms of one or more included features and/or operation: the extension set 10 of Figures 1A-1C, the extension set 52 of Figures 2A-2C, the extension set 56 of Figures 3A-3C, and the extension set 70 of Figures 4A-4C.

In some embodiments, in response to the second luer adapter 24 being tightened with respect to the third luer adapter 34, a first fluid pathway 74 through the first extension tube 58 may be open, and a second fluid pathway 76 through the second extension tube 60 may be closed. In some embodiments, in response to the second luer adapter 24 being loosened with respect to the third luer adapter 34, the first fluid pathway 74 through the first extension tube 58 may be open, and the second fluid pathway 76 through the second extension tube 60 may be open. In some embodiments, to decrease the flow resistance within the extension set 72 after the blood collection container is partially filled and the pressure differential has decreased, the clinician may loosen the second luer adapter 24 with respect to the third luer adapter 34 or move the second luer adapter 24 from a tightened position to a loosened position. In some embodiments, the second luer adapter 24 and the third luer adapter 34 may include corresponding threads, and the second luer adapter 24 may be unthreaded with respect to the third luer adapter 34 to move the second luer adapter 24 from the tightened position to the loosened position.

In some embodiments, the extension set 72 may include a third extension tube 78, which may be integrated with the third luer adapter 34. In some embodiments, the third luer adapter 34 may include one or more openings 80, each in fluid communication with a particular extension tube. In some embodiments, the opening 80 in fluid communication with the first extension tube 58 may remain open in response to the second luer adapter 24 being in the loosened position and the tightened position with respect to the third luer adapter 34.

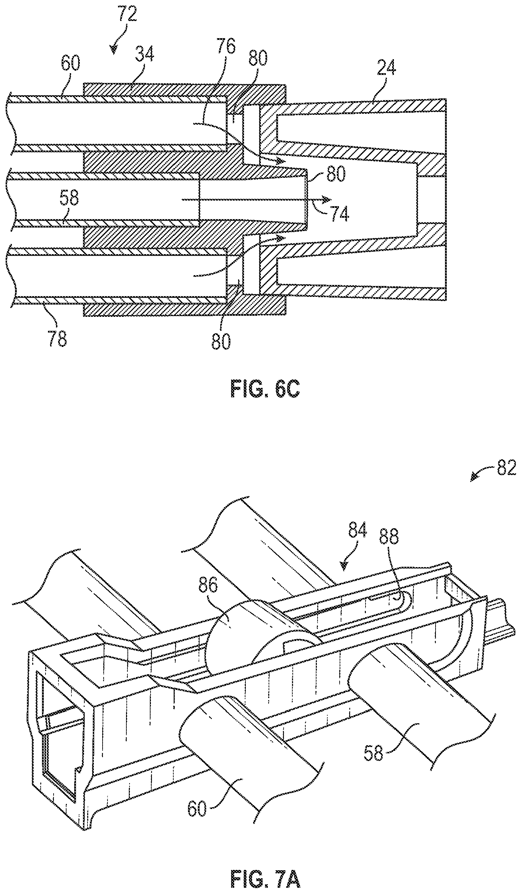

In some embodiments, the openings 80 in fluid communication with the second extension tube 60 and/or the third extension tube 78 may be blocked by the second luer adapter 24 when the second luer adapter 24 is in the tightened position with respect to the third luer adapter 34, as illustrated, for example, in Figure 6B. In some embodiments, the openings 80 in fluid communication with the second extension tube 60 and/or the third extension tube 78 may be unblocked in response to the second luer adapter 24 being in the loosened position with respect to the third luer adapter 34, as illustrated, for example, in Figure 6C. In these embodiments, the second luer adapter 24 may be spaced apart from the openings 80 in fluid communication with the second extension tube 60 and/or the third extension tube 78.

Referring now to Figures 7A-7C, a portion of an extension set 82 is illustrated, according to some embodiments. In some embodiments, the extension set 82 may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, and the extension set 72 of Figures 6A-6C.

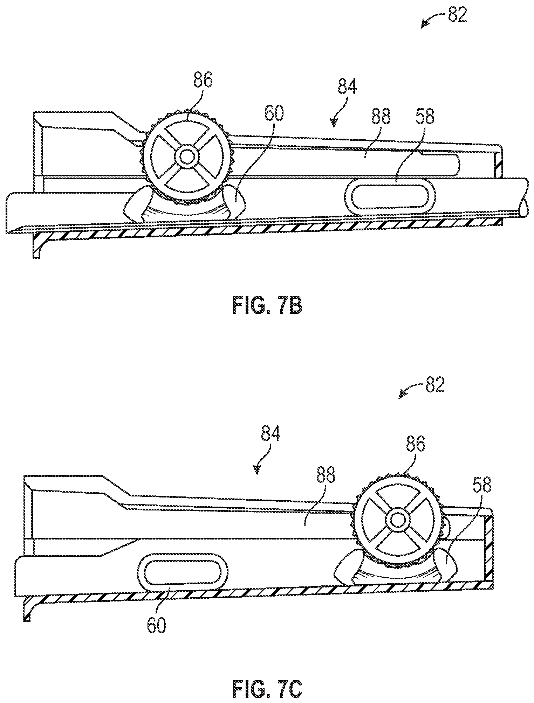

In some embodiments, the extension set 82 may include a roller clamp 84. In some embodiments, the roller clamp 84 may include a roller 86 moveable within a track 88. In some embodiments, the first extension tube 58 and the second extension tube 60 may extend through the roller clamp 84 generally perpendicular to the track 88. In some embodiments, when blood flow through the second extension tube 60 is blocked, the roller 86 may be disposed in a first position proximate the second extension tube 60, as illustrated, for example, in Figure 7B, and may pinch the second extension tube 60. In some embodiments, opening the second extension tube 60 may include moving the roller 86 along the track 88 away from the second extension tube 60 and toward the first extension tube 58. In some embodiments, when blood flow through the first extension tube 58 is blocked, the roller 86 may be disposed in a second position proximate the first extension tube 58, as illustrated, for example, in Figure 7C, and may pinch the first extension tube 58.

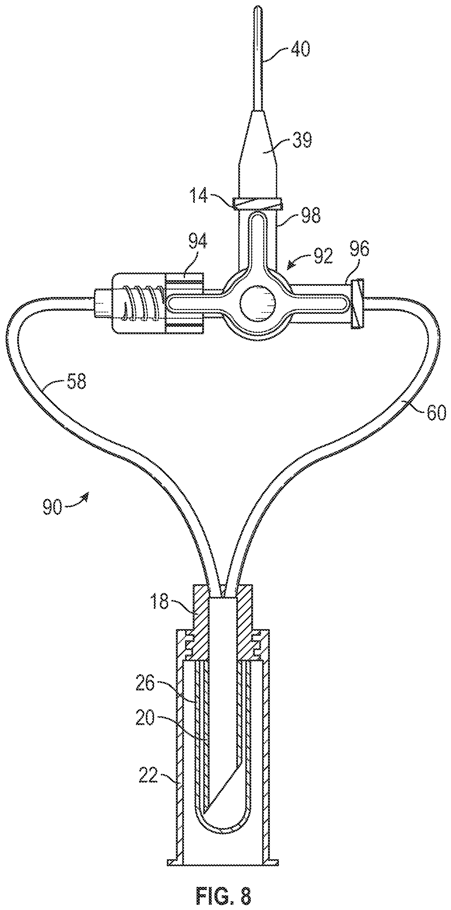

Referring now to Figure 8, an extension set 90 is illustrated, according to some embodiments. In some embodiments, the extension set 90 may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, and the extension set 82 of Figures 7A-7C.

In some embodiments, the distal end 12 of the extension set 90 may include a three-way stopcock 92, which may include a first port 94, a second port 96, and a third port 98. In some embodiments, the first extension tube 58 may be coupled with the first port 94, the second extension tube 60 may be coupled to the second port 96, and the third port 98 may include the first luer adapter 14 configured to couple to the catheter adapter 39. In some embodiments, blocking blood flow through the second extension tube 60 may include rotating the three-way stopcock 92 to a first position. In some embodiments, opening the second extension tube 60 may include rotating the three-way stopcock 92 to a second position.

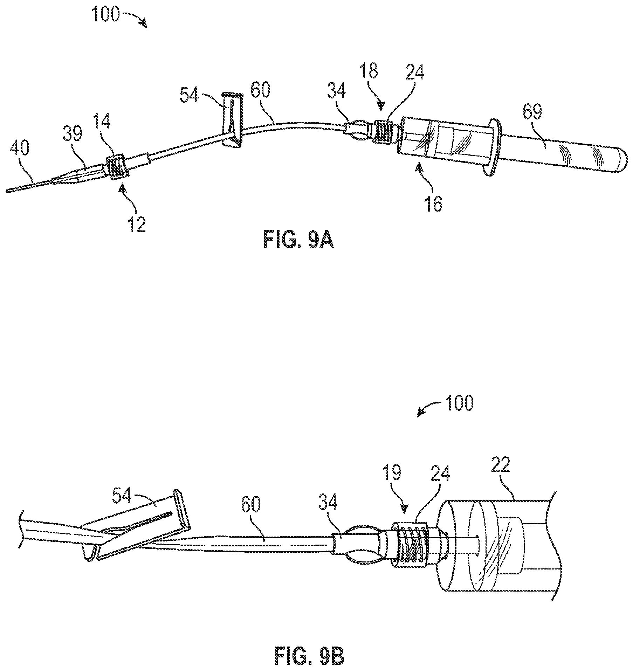

Referring now to Figures 9A-9E, an extension set 100 is illustrated, according to some embodiments. In some embodiments, the extension set 100 may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8.

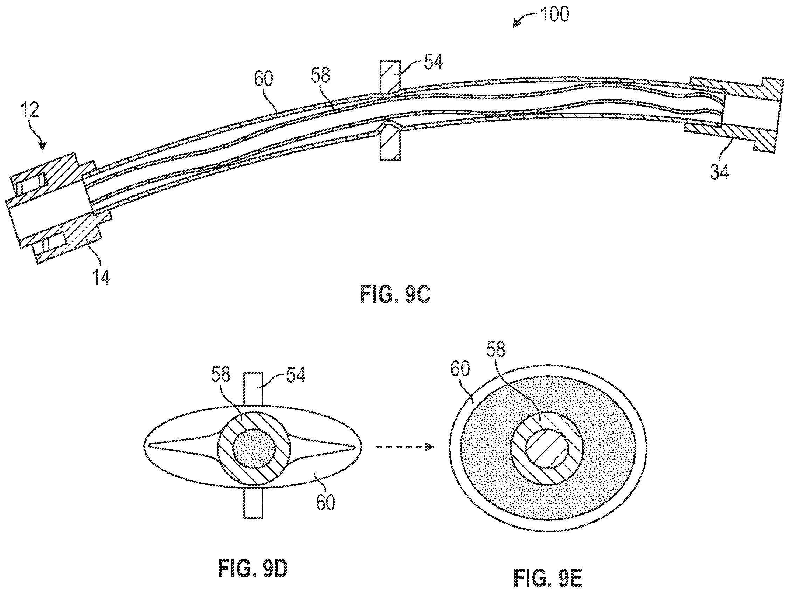

In some embodiments, the first extension tube 58 may be disposed within the second extension tube 60. In some embodiments, in response to the clamp 54 being in the clamped position, blood may flow through the first extension tube 58, and blood flow between an outer surface of the first extension tube 58 and an inner surface of the second extension tube 60 may decrease, as illustrated, for example, in Figure 9D. In some embodiments, to increase the flow resistance within the catheter system after the blood collection container 69 is partially filled and the pressure differential has decreased, the clinician may move the clamp 54 to the unclamped position such that blood flows between the outer surface of the first extension tube 58 and the inner surface of the second extension tube 60, as illustrated, for example, in Figure 9E. In some embodiments, the first extension tube 58 may be longer than the second extension tube 60 and/or an inner diameter of the first extension tube 58 may be less than an inner diameter of the second extension tube 60.

In some embodiments, the first extension tube 58 may be configured to collapse at a different pressure differential than the second extension tube 60. In some embodiments, the second extension tube 60 may include a lower durometer than the first extension tube 58. In some embodiments, the second extension tube 60 may be configured to collapse at a lower pressure differential than the first extension tube 58. In some embodiments, at the lower pressure differential, the second extension tube 60 may contact the first extension tube 58 to reduce or stop blood flow between the outer surface of the first extension tube 58 and the inner surface of the second extension tube 60. In some embodiments, in response to the blood collection container 18 partially filling with blood, the blood flow between the outer surface of the first extension tube 58 and the inner surface of the second extension tube 60 may increase.

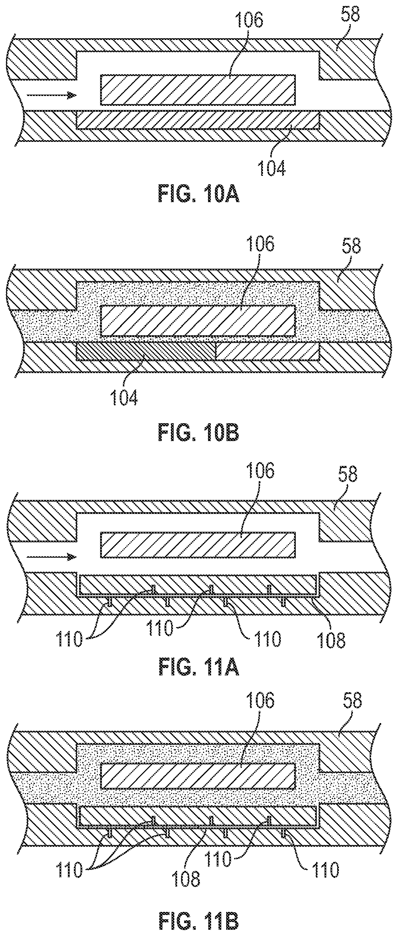

Referring now to Figures 10A-10B, in some embodiments, an inner surface of the first extension tube 58 may include an absorbent material 104. In some embodiments, blood within the first extension tube 58 may be configured to flow proximate the absorbent material 104. In some embodiments, in response to blood reaching or saturating a particular portion of the absorbent material 104, as illustrated, for example in Figure 10B, or an entirety of the absorbent material 104, the clinician may change the flow resistance via a particular extension set. In some embodiments, the particular extension set may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8.

In some embodiments, the first fluid pathway 74 through the first extension tube 58 may be narrowed proximate the absorbent material 104, such as through an island 106, which may be disposed within the first fluid pathway 74. In some embodiments, blood may flow around the island 106 in a distal to proximal direction, as illustrated, for example, in Figure 10B.

Referring now to Figures 11A-11B, in some embodiments, the first extension tube 58 may include an indicator pathway or tube 108, which may include a first end and a second end connected to a primary portion of the first fluid pathway 74 of the first extension tube 58. In some embodiments, blood within the first extension tube 58 may be configured to flow through the indicator tube 108, which may include a smaller diameter than the primary portion of the first fluid pathway 74.

In some embodiments, the indicator tube 108 may include one or more markers 110, which may include grooves configured to collect blood or lines. In some embodiments, the markers 110 may provide a visual indicator to the clinician. In some embodiments, in response to blood reaching a particular marker 110, as illustrated, for example in Figure 11B, or all of the markers 110, the clinician may change the flow resistance via a particular extension set. In some embodiments, the particular extension set may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8.

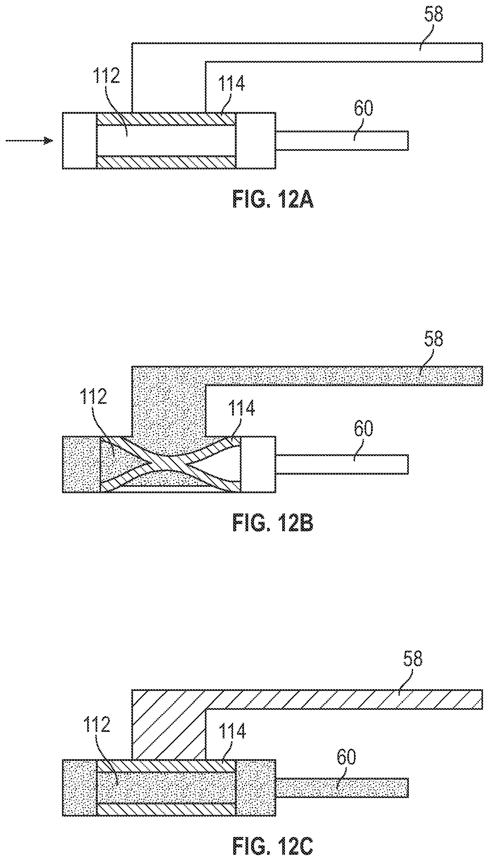

Referring now to Figures 12A-12C, in some embodiments, the first extension tube 58 and the second extension tube 60 may join at a junction 112. In some embodiments, the junction 112 may be disposed within the first luer adapter 14, proximal to the first luer adapter 14, or at another location within a particular extension set. In some embodiments, the particular extension set may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8.

In some embodiments, the particular extension set may include a pressure-sensitive valve 114. In some embodiments, the pressure-sensitive valve 114 may be disposed at a junction of the first fluid pathway 74 and the second fluid pathway 76. In some embodiments, the pressure-sensitive valve 114 may be disposed in the second extension tube 60. In some embodiments, the pressure-sensitive valve 114 may be disposed in a portion of the fluid pathway of the particular extension set distal to the first extension tube 58 and the second extension tube 60. In some embodiments, the fluid pathway of the particular extension set may extend through the proximal end 16 and the distal end 12. In some embodiments, the fluid pathway of the particular extension set may include the first fluid pathway 74 and/or the second fluid pathway 76.

In some embodiments, in response to a first predetermined pressure differential within the particular extension set, the pressure-sensitive valve 114 may be open with respect to the first extension tube 58 and closed with respect to the second extension tube 60. In some embodiments, in response to a second predetermined pressure differential within the particular extension set, the pressure-sensitive valve 114 may remain open with respect to the first extension tube 58 and open with respect to the second extension tube 60. In some embodiments, the first predetermined pressure differential may be greater than the second predetermined pressure differential. In some embodiments, the first predetermined pressure differential and the second predetermined pressure differential may correspond to the pressure differential between the vein of the patient and the blood collection container coupled to the particular extension set. In some embodiments, the pressure-sensitive valve 114 may include any suitable type of pressure-sensitive valve. In some embodiments, an inner surface of the particular extension set may include one or more grooves proximate the pressure-sensitive valve. In some embodiments, the grooves may allow blood to bypass the pressure-sensitive valve 114 and flow into the first extension tube 58 but not the second extension tube 60.

In some embodiments, a not claimed method of blood collection or collecting a blood sample may include inserting a catheter of a catheter system into vasculature of the patient. In some embodiments, the catheter system may include a catheter assembly, which may include a catheter adapter and the catheter. In some embodiments, the catheter may be secured within the catheter adapter and may extend distally from the catheter adapter.

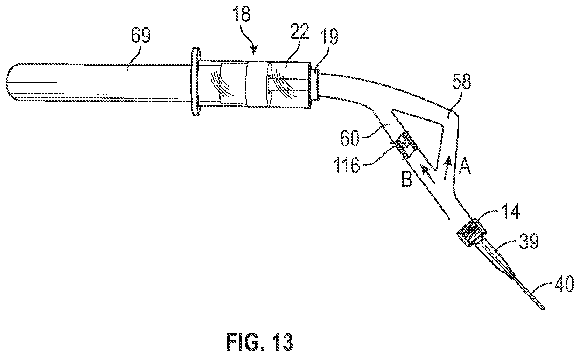

Referring now to Figure 13, in some embodiments, a pressure-sensitive valve 116 may be disposed within the second extension tube 60, which may be shorter than the first extension tube 58 and/or include a larger inner diameter than the first extension tube 58 In some embodiments, the pressure-sensitive valve 116 may include a duckbill valve, as illustrated, for example, in Figure 13, or another suitable type of pressure-sensitive valve. For example, the pressure-sensitive valve 116 may include a spring-loaded plunger or ball. In some embodiments, the second extension tube 60 may be more compliant than the first extension tube 58 and thus may close at a lower pressure differential than the first extension tube 58.

In some embodiments, in response to a first predetermined pressure differential within a particular extension set, the pressure-sensitive valve 116 may be closed and not allow blood to flow through the second extension tube 60. In some embodiments, in response to a second predetermined pressure differential within the particular extension set, the pressure-sensitive valve 116 may open. In some embodiments, the first predetermined pressure differential may be greater than the second predetermined pressure differential. In some embodiments, the particular extension set may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8.

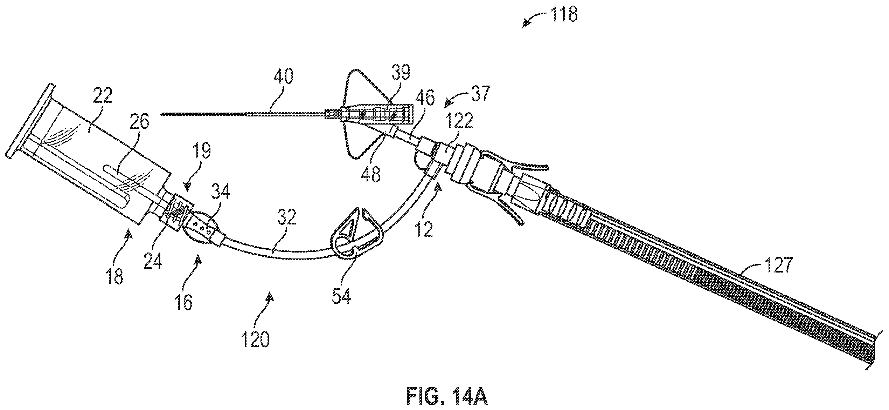

Referring now to Figure 14A, a catheter system 118 is illustrated, according to some embodiments. In some embodiments, the catheter system 118 may include an extension set 120, which may be integrated with the catheter assembly 37. In further detail, in some embodiments, the distal end 12 of the extension set 120 may be integrated with an adapter 122 of the catheter assembly 37, as illustrated, for example, in Figure 14A, or integrated with the catheter adapter 39 itself. In these and other embodiments, the extension set 120 may not be removable from the catheter system 118. In some embodiments, the distal end of the first extension tube 58 or the distal end of the extension tube 32 may be integrated with the adapter 122 or the catheter adapter 39. In some embodiments, the adapter 122 may include a Y-adapter, a T-adapter, or another suitable adapter.

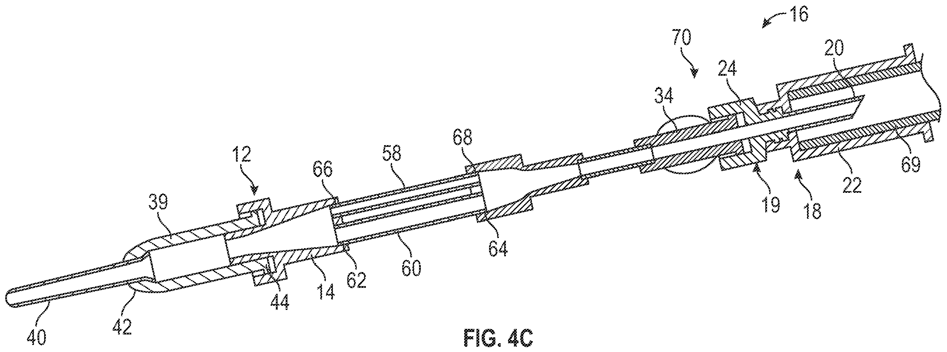

In some embodiments, the first extension tube 58 and/or the second extension tube 60 (see, for example, Figures 4-9) may be integrated into the side port 48. In some embodiments, the other extension tube 46 may be similar or identical to the first extension tube 58 in terms of one or more included features and/or operation. In some embodiments, the extension set 120 may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8. In some embodiments, the extension set 120 may be similar or identical to one or more of the following in terms of one or more included features and/or operation: the extension sets of Figures 9-13.

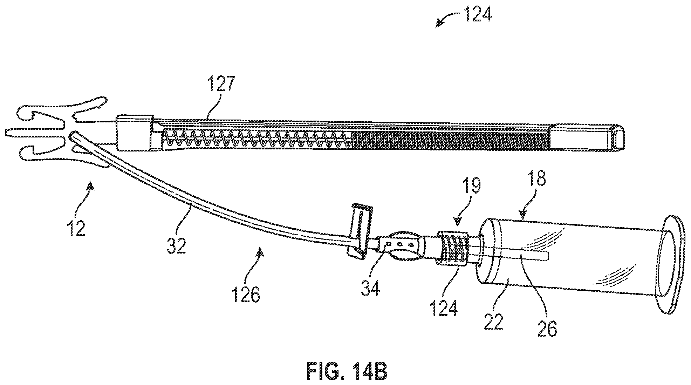

Referring now to Figure 14B, a catheter system 124 is illustrated, according to some embodiments. In some embodiments, the catheter system 124 may include an extension set 126, which may be coupled to or integrated with an instrument delivery device 127, which may deliver a probe, a catheter, or a guidewire through a particular catheter assembly (as illustrated, for example, in Figure 14A). In some embodiments, the first extension tube 58 and/or the second extension tube 60 (see, for example, Figures 4-9) may be integrated into the instrument delivery device 127.

In some embodiments, the extension set 126 may be similar or identical to one or more of the following in terms of one or more included features and/or operation: the extension set 10 of Figures 1A-1C, the extension set 52 of Figures 2A-2C, the extension set 56 of Figures 3A-3C, the extension set 70 of Figures 4A-4C, the extension set 72 of Figures 6A-6C, the extension set 82 of Figures 7A-7C, and the extension set 90 of Figure 8. In some embodiments, the extension set 126 may be similar or identical to one or more of the following in terms of one or more included features and/or operation: the extension sets of Figures 9-13.

In some embodiments, the instrument delivery device 127 may include any suitable instrument delivery device. In some embodiments, the instrument delivery device 127 may be further described in U.S. Patent Application No. 16/037,246, filed July 17, 2018, entitled "EXTENSION HOUSING A PROBE OR INTRAVENOUS CATHETER," U.S. Patent Application No 16/388,650, filed April 18, 2019, entitled "INSTRUMENT DELIVERY DEVICE HAVING A ROTARY ELEMENT," U.S. Patent Application No. 16/037,319, filed July 17, 2018, entitled "MULTI-DIAMETER CATHETER AND RELATED DEVICES AND METHODS," U.S. Patent Application No. 16/502,541, filed July 3, 2019, entitled "DELIVERY DEVICE FOR A VASCULAR ACCESS INSTRUMENT," U.S. Patent Application No. 16/691,217, filed November 21, 2019, entitled "SYRINGE-BASED DELIVERY DEVICE FOR A VASCULAR ACCESS INSTRUMENT," U.S. Patent Application No. 62/794,437, filed January 18, 2019, entitled "CATHETER DELIVERY DEVICE AND RELATED SYSTEMS AND METHODS," and U.S. Patent Application No. 62/830,286, filed April 5, 2019, entitled "VASCULAR ACCESS INSTRUMENT HAVING A FLUID PERMEABLE STRUCTURE AND RELATED DEVICES AND METHODS," entirety.

**Claims**

1. A catheter extension set (10, 52), comprising:

   a distal end (12), comprising a luer adapter (14) configured to couple to a catheter adapter (39); a proximal end (16), comprising a blood collection device (18); and an extension tube (32) extending between the distal end (12) and the proximal end (16), **characterized in that** an inner surface of the extension tube (32) comprises an absorbent material (104) or an indicator tube (108) comprising a plurality of markers (110), wherein blood within the extension tube (32) is configured to flow proximate the absorbent material (104) or through the indicator tube (108).

2. The catheter extension set (10, 52) of claim 1, further comprising a clamp (54) disposed on the extension tube (32), wherein the clamp (54) is configured to move between a clamped position and an unclamped position.

3. The catheter extension set (10, 52) of claim 1, wherein the luer adapter (14) is a first luer adapter (14), wherein the blood collection device (18) comprises a second luer adapter (24), further comprising a third luer adapter (34) coupled to the second luer adapter (24), wherein the extension tube (32) comprises a distal end (36) and a proximal end (38), wherein the distal end (36) of the extension tube (32) is integrated with the first luer adapter (14), wherein the proximal end (38) of the extension tube (32) is integrated with the third luer adapter (34).

4. The catheter extension set (10, 52) of claim 1, wherein the extension tube (32) comprises a distal end (36) and a proximal end (38), wherein the distal end (36) of the extension tube (32) is integrated with the luer adapter (14), wherein the proximal end (38) of the extension tube (32) is integrated with the blood collection device (18).

5. A catheter extension set (56, 70, 90, 100), comprising:

   a distal end (12), comprising a luer adapter (14) configured to couple to a catheter adapter (39); a proximal end (16), comprising a blood collection device (18); a first extension tube (58) extending between the distal end (12) and the proximal end (16); and a second extension tube (60) extending between the distal end (12) and the proximal end (16), **characterized in that** an inner surface of the first extension tube (58) and/or the second extension tube (60) comprises an absorbent material (104) or an indicator tube (108) comprising a plurality of markers (110), wherein blood within the first extension tube (58) and/or the second extension tube (60) is configured to flow proximate the absorbent material (104) or through the indicator tube (108).

6. The catheter extension set (56, 70, 90, 100) of claim 5, further comprising a clamp (54, 84), wherein the second extension tube (60) extends through the clamp (54, 84), wherein the clamp (54, 84) is configured to move between a clamped position and an unclamped position.

7. The catheter extension set (56, 70, 90, 100) of claim 6, wherein the first extension tube (58) is longer than the second extension tube (60).

8. The catheter extension set (56, 70, 90, 100) of claim 6, wherein an inner diameter of the first extension tube (58) is less than an inner diameter of the second extension tube (60).

9. The catheter extension set (100) of claim 6, wherein the first extension tube (58) is disposed within the second extension tube (60), wherein in response to the clamp (54) being in the clamped position, blood flows through the first extension tube (58) and blood flow between an outer surface of the first extension tube (58) and an inner surface of the second extension tube (60) is decreased.

10. The catheter extension set (56, 70) of claim 5, wherein the luer adapter (14) is a first luer adapter (14), wherein the blood collection device (18) comprises a second luer adapter (24), further comprising a third luer adapter (34) coupled to the second luer adapter (24), wherein the first extension tube (58) comprises a distal end (66) and a proximal end (68), wherein the second extension tube (60) comprises a distal end (62) and a proximal end (64), wherein the distal end (66) of the first extension tube (58) and the distal end (62) of the second extension tube (60) are integrated with the first luer adapter (14), wherein the proximal end (68) of the first extension tube (58) and the proximal end (64) of the second extension tube (60) are integrated with the third luer adapter (34), wherein in response to the second luer adapter (24) being tightened with respect to the third luer adapter (34), a first fluid pathway (74) through the first extension tube (58) is open and a second fluid pathway (76) through the second extension tube (60) is closed, wherein in response to the second luer adapter (24) being loosened with respect to the third luer adapter (34), the first fluid pathway (74) through the first extension tube (58) is open and the second fluid pathway (76) through the second extension tube (60) is open.

11. The catheter extension set (56, 70, 90, 100) of claim 5, further comprising a pressure-sensitive valve (114) within a fluid pathway of the catheter extension set distal to the first extension tube (58) and the second extension tube (60), wherein in response to a first predetermined vacuum pressure level within the catheter extension set, the pressure-sensitive valve (114) is open with respect to the first extension tube (58) and closed with respect to the second extension tube (60), wherein in response to a second predetermined vacuum pressure level within the catheter extension set, the pressure-sensitive valve (114) is closed with respect to the first extension tube and open with respect to the second extension tube, wherein the first predetermined vacuum pressure level is greater than the second predetermined vacuum pressure level.

12. The catheter extension set of claim 5, wherein the second extension tube (60) comprises a pressure-sensitive valve (116).

**Patentansprüche**

1. Katheterverlängerungssatz (10, 52), der aufweist:

ein distales Ende (12), das einen Luer-Adapter (14) aufweist, der dazu ausgebildet ist, sich mit einem Katheteradapter (39) zu verbinden; ein proximales Ende (16), das eine Blutentnahmevorrichtung (18) aufweist; und einen Verlängerungsschlauch (32), der sich zwischen dem distalen Ende (12) und dem proximalen Ende (16) erstreckt, **dadurch gekennzeichnet, dass** eine Innenfläche des Verlängerungsschlauchs (32) ein absorbierendes Material (104) oder ein Anzeigeröhrchen (108) aufweist, das eine Vielzahl von Markierungen (110) hat, wobei das Blut in dem Verlängerungsschlauch (32) in der Nähe des absorbierenden Materials (104) oder durch das Anzeigeröhrchen (108) fließen soll.

2. Katheterverlängerungssatz (10, 52) nach Anspruch 1, der ferner eine Klemme (54) aufweist, die sich an dem Verlängerungsschlauch (32) befindet, wobei die Klemme (54) so ausgebildet ist, dass sie sich zwischen einer gespannten Position und einer ungespannten Position bewegen kann.

3. Katheterverlängerungssatz (10, 52) nach Anspruch 1, wobei der Luer-Adapter (14) ein erster Luer-Adapter (14) ist, wobei die Blutentnahmevorrichtung (18) einen zweiten Luer-Adapter (24) aufweist, der ferner einen mit dem zweiten Luer-Adapter (24) gekoppelten dritten Luer-Adapter (34) aufweist, wobei der Verlängerungsschlauch (32) ein distales Ende (36) und ein proximales Ende (38) aufweist, wobei das distale Ende (36) des Verlängerungsschlauchs (32) mit dem ersten Luer-Adapter (14) integriert ist, wobei das proximale Ende (38) des Verlängerungsschlauchs (32) mit dem dritten Luer-Adapter (34) integriert ist.

4. Katheterverlängerungssatz (10, 52) nach Anspruch 1, wobei der Verlängerungsschlauch (32) ein distales Ende (36) und ein proximales Ende (38) aufweist, wobei das distale Ende (36) des Verlängerungsschlauchs (32) mit dem Luer-Adapter (14) integriert ist, wobei das proximale Ende (38) des Verlängerungsschlauchs (32) mit der Blutentnahmevorrichtung (18) integriert ist.

5. Katheterverlängerungssatz (56, 70, 90, 100), der aufweist:

ein distales Ende (12), das einen Luer-Adapter (14) aufweist, der dazu ausgebildet ist, sich mit einem Katheteradapter (39) zu verbinden;
ein proximales Ende (16), das eine Blutentnahmevorrichtung (18) aufweist;
einen ersten Verlängerungsschlauch (58), der sich zwischen dem distalen Ende (12) und dem proximalen Ende (16) erstreckt; und
einen zweiten Verlängerungsschlauch (60), der sich zwischen dem distalen Ende (12) und dem proximalen Ende (16) erstreckt,
**dadurch gekennzeichnet, dass** eine Innenfläche des ersten Verlängerungsschlauchs (58) und/oder des zweiten Verlängerungsschlauchs (60) ein absorbierendes Material (104) oder ein Anzeigeröhrchen (108) aufweist, das eine Vielzahl von Markierungen (110) hat, wobei das Blut in dem ersten Verlängerungsschlauch (58) und/oder dem zweiten Verlängerungsschlauch (60) in der Nähe des absorbierenden Materials (104) oder durch das Anzeigeröhrchen (108) fließen soll.

6. Katheterverlängerungssatz (56, 70, 90, 100) nach Anspruch 5, der ferner eine Klemme (54, 84) aufweist, wobei sich der zweite Verlängerungsschlauch (60) durch die Klemme (54, 84) erstreckt, wobei die Klemme (54, 84) so ausgebildet ist, dass sie sich zwischen einer gespannten Position und einer ungespannten Position bewegt.

7. Katheterverlängerungssatz (56, 70, 90, 100) nach Anspruch 6, wobei der ersten Verlängerungsschlauch (58) länger als der zweite Verlängerungsschlauch (60) ist.

8. Katheterverlängerungssatz (56, 70, 90, 100) nach Anspruch 6, wobei ein Innendurchmesser des ersten Verlängerungsschlauchs (58) kleiner als ein Innendurchmesser des zweiten Verlängerungsschlauchs (60) ist.

9. Katheterverlängerungssatz (100) nach Anspruch 6, wobei der erste Verlängerungsschlauch (58) in dem zweiten Verlängerungsschlauch (60) angeordnet ist, wobei als Reaktion darauf, dass sich die Klemme (54) in der gespannten Position befindet, das Blut durch den ersten Verlängerungsschlauch (58) fließt und der Blutfluss zwischen einer Außenfläche des ersten Verlängerungsschlauchs (58) und einer Innenfläche des zweiten Verlängerungsschlauchs (60) abnimmt.

10. Katheterverlängerungssatz (56, 70) nach Anspruch 5, wobei der Luer-Adapter (14) ein erster Luer-Adapter (14) ist, wobei die Blutentnahmevorrichtung (18) einen zweiten Luer-Adapter (24) aufweist, der ferner einen mit dem zweiten Luer-Adapter (24) gekoppelten dritten Luer-Adapter (34) aufweist, wobei der erste Verlängerungsschlauch (58) ein distales Ende (66) und ein proximales Ende (68) aufweist, wobei der zweite Verlängerungsschlauch (60) ein distales Ende (62) und ein proximales Ende (64) aufweist, wobei das distale Ende (66) des ersten Verlängerungsschlauchs (58) und das distale Ende des zweiten Verlängerungsschlauchs (60) mit dem ersten Luer-Adapter (14) integriert sind, wobei das proximale Ende (68) des ersten Verlängerungsschlauchs (58) und das proximale Ende (64) des zweiten Verlängerungsschlauchs (60) mit dem dritten Luer-Adapter (34) integriert sind, wobei als Reaktion darauf, dass der zweite Luer-Adapter (24) in Bezug auf den dritten Luer-Adapter (34) angezogen ist, ein erster Flüssigkeitsweg (74) durch den ersten Verlängerungsschlauch (58) geöffnet ist und ein zweiter Flüssigkeitsweg (76) durch den zweiten Verlängerungsschlauch (60) geschlossen ist, wobei als Reaktion darauf, der zweite Luer-Adapter (24) in Bezug auf den dritten Luer-Adapter (34) gelockert ist, der erste Flüssigkeitsweg (74) durch den ersten Verlängerungsschlauch (58) geöffnet ist und der zweite Flüssigkeitsweg (76) durch den zweiten Katheterverlängerungssatz (60) geöffnet ist.

11. Katheterverlängerungssatz (56, 70, 90, 100) nach Anspruch 5, der ferner ein druckempfindliches Ventil (114) in einem Flüssigkeitsweg des Katheterverlängerungssatzes distal zum ersten Verlängerungsschlauch (58) und zum zweiten Verlängerungsschlauch (60) aufweist, wobei als Reaktion auf ein erstes vorbestimmtes Vakuumdruckniveau in dem Katheterverlängerungssatz das druckempfindliche Ventil (114) in Bezug auf den ersten Verlängerungsschlauch (58) geöffnet ist und in Bezug auf den zweiten Verlängerungsschlauch (60) geschlossen ist, wobei als Reaktion auf ein zweites vorbestimmten Vakuumdruckniveau in dem Katheterverlängerungssatz das druckempfindliche Ventil (114) in Bezug auf den ersten Verlängerungsschlauch geschlossen ist und in Bezug auf den zweiten Verlängerungsschlauch geöffnet ist, wobei das erste vorbestimmte Vakuumdruckniveau größer als das zweite vorbestimmte Vakuumdruckniveau ist.

12. Katheterverlängerungssatz nach Anspruch 5, wobei der zweite Verlängerungsschlauch (60) ein druckempfindliches Ventil (116) aufweist.

**Revendications**

1. Ensemble extension de cathéter (10, 52), comprenant :

une extrémité distale (12), comprenant un adaptateur Luer (14) configuré pour se coupler à un adaptateur de cathéter (39) ;
une extrémité proximale (16), comprenant un dispositif de collecte de sang (18) ; et
un tube d'extension (32) s'étendant entre l'extrémité distale (12) et l'extrémité proximale (16), **caractérisé en ce qu'**une surface interne du tube d'extension (32) comprend un matériau absorbant (104) ou un tube indicateur (108) comprenant une pluralité de marqueurs (110), dans lequel le sang à l'intérieur du tube d'extension (32) est configuré pour s'écouler à proximité du matériau absorbant (104) ou à travers le tube indicateur (108).

2. Ensemble extension de cathéter (10, 52) de la revendication 1, comprenant en outre une pince (54) disposée sur le tube d'extension (32), dans lequel la pince (54) est configurée pour se déplacer entre une position serrée et une position desserrée.

3. Ensemble extension de cathéter (10, 52) de la revendication 1, dans lequel l'adaptateur Luer (14) est un premier adaptateur Luer (14), dans lequel le dispositif de collecte de sang (18) comprend un deuxième adaptateur Luer (24), comprenant en outre un troisième adaptateur Luer (34) couplé au deuxième adaptateur Luer (24), dans lequel le tube d'extension (32) comprend une extrémité distale (36) et une extrémité proximale (38), dans lequel l'extrémité distale (36) du tube d'extension (32) est intégrée au premier adaptateur Luer (14), dans lequel l'extrémité proximale (38) du tube d'extension (32) est intégrée au troisième adaptateur Luer (34).

4. Ensemble extension de cathéter (10, 52) de la revendication 1, dans lequel le tube d'extension (32) comprend une extrémité distale (36) et une extrémité proximale (38), dans lequel l'extrémité distale (36) du tube d'extension (32) est intégrée à l'adaptateur Luer (14), dans lequel l'extrémité proximale (38) du tube d'extension (32) est intégrée au dispositif de collecte de sang (18).

5. Ensemble extension de cathéter (56, 70, 90, 100), comprenant :

une extrémité distale (12), comprenant un adaptateur Luer (14) configuré pour se coupler à un adaptateur de cathéter (39) ;
une extrémité proximale (16), comprenant un dispositif de collecte de sang (18) ;
un premier tube d'extension (58) s'étendant entre l'extrémité distale (12) et l'extrémité proximale (16) ; et
un deuxième tube d'extension (60) s'étendant entre l'extrémité distale (12) et l'extrémité proximale (16), **caractérisé en ce qu'**une surface interne du premier tube d'extension (58) et/ou du deuxième tube d'extension (60) comprend un matériau absorbant (104) ou un tube indicateur (108) comprenant une pluralité de marqueurs (110), dans lequel le sang à l'intérieur du premier tube d'extension (58) et/ou du deuxième tube d'extension (60) est configuré pour s'écouler à proximité du matériau absorbant (104) ou à travers le tube indicateur (108).

6. Ensemble extension de cathéter (56, 70, 90, 100) de la revendication 5, comprenant en outre une pince (54, 84), dans lequel le deuxième tube d'extension (60) s'étend à travers la pince (54, 84), dans lequel la pince (54, 84) est configurée pour se déplacer entre une position serrée et une position desserrée.

7. Ensemble extension de cathéter (56, 70, 90, 100) de la revendication 6, dans lequel le premier tube d'extension (58) est plus long que le deuxième tube d'extension (60).

8. Ensemble extension de cathéter (56, 70, 90, 100) de la revendication 6, dans lequel un diamètre intérieur du premier tube d'extension (58) est inférieur à un diamètre intérieur du deuxième tube d'extension (60).

9. Ensemble extension de cathéter (100) de la revendication 6, dans lequel le premier tube d'extension (58) est disposé à l'intérieur du deuxième tube d'extension (60), dans lequel en réponse au fait que la pince (54) est dans la position serrée, le sang s'écoule à travers le premier tube d'extension (58) et l'écoulement de sang entre une surface externe du premier tube d'extension (58) et une surface interne du deuxième tube d'extension (60) est diminué.

**10.** Ensemble extension de cathéter (56, 70) de la revendication 5, dans lequel l'adaptateur Luer (14) est un premier adaptateur Luer (14), dans lequel le dispositif de collecte de sang (18) comprend un deuxième adaptateur Luer (24), comprenant en outre un troisième adaptateur Luer (34) couplé au deuxième adaptateur Luer (24), dans lequel le premier tube d'extension (58) comprend une extrémité distale (66) et une extrémité proximale (68), dans lequel le deuxième tube d'extension (60) comprend une extrémité distale (62) et une extrémité proximale (64), dans lequel l'extrémité distale (66) du premier tube d'extension (58) et l'extrémité distale (62) du deuxième tube d'extension (60) sont intégrées au premier adaptateur Luer (14), dans lequel l'extrémité proximale (68) du premier tube d'extension (58) et l'extrémité proximale (64) du deuxième tube d'extension (60) sont intégrées au troisième adaptateur Luer (34), dans lequel, en réponse au serrage du deuxième adaptateur Luer (24) par rapport au troisième adaptateur Luer (34), un premier chemin de fluide (74) à travers le premier tube d'extension (58) est ouvert et un deuxième chemin de fluide (76) à travers le deuxième tube d'extension (60) est fermé, dans lequel en réponse au desserrage du deuxième adaptateur Luer (24) par rapport au troisième adaptateur Luer (34), le premier chemin de fluide (74) à travers le premier tube d'extension (58) est ouvert et le deuxième chemin de fluide (76) à travers le deuxième tube d'extension (60) est ouvert.

**11.** Ensemble extension de cathéter (56, 70, 90, 100) de la revendication 5, comprenant en outre une valve sensible à la pression (114) dans un chemin de fluide de l'ensemble extension de cathéter distal par rapport au premier tube d'extension (58) et au deuxième tube d'extension (60), dans lequel en réponse à un premier niveau de pression de vide prédéterminé à l'intérieur de l'ensemble extension de cathéter, la valve sensible à la pression (114) est ouverte par rapport au premier tube d'extension (58) et fermée par rapport au deuxième tube d'extension (60), dans lequel en réponse à un deuxième niveau de pression de vide prédéterminé à l'intérieur de l'ensemble extension de cathéter, la valve sensible à la pression (114) est fermée par rapport au premier tube d'extension et ouverte par rapport au deuxième tube d'extension, dans lequel le premier niveau de pression de vide prédéterminé est supérieur au deuxième niveau de pression de vide prédéterminé.

**12.** Ensemble extension de cathéter de la revendication 5, dans lequel le deuxième tube d'extension (60) comprend une valve sensible à la pression (116).

FIG. 1A

FIG. 1B

FIG. 1C

EP 4 076 188 B1

FIG. 2A

FIG. 2B

20

FIG. 2C

FIG. 3A

FIG. 3B

EP 4 076 188 B1

FIG. 3C

FIG. 4A

FIG. 4B

FIG. 4C

EP 4 076 188 B1

58

Blood →                    → Blood Collection Container

60
54
FIG. 5A

58

60
54   FIG. 5B

58

60   FIG. 5C

26

FIG. 6A

FIG. 6B

FIG. 6C

FIG. 7A

FIG. 7B

FIG. 7C

FIG. 8

**FIG. 9A**

**FIG. 9B**

FIG. 9C

FIG. 9D

FIG. 9E

EP 4 076 188 B1

FIG. 10A

FIG. 10B

FIG. 11A

FIG. 11B

FIG. 12A

FIG. 12B

FIG. 12C

FIG. 13

FIG. 14A

FIG. 14B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019236956 A2
- US 6292869 A
- US 03724618
- US 38865019
- US 03731918
- US 50254119
- US 69121719
- US 62794437
- US 62830286